# EUROPEAN PATENT APPLICATION

(11) **EP 4 000 456 A1**
(43) Date of publication of application: **25.05.2022**
(21) Application number: 20209454.6
(22) Date of filing: 24.11.2020
(51) Int. Cl.: A46B 15/00, A61C 17/22

(54) **SYSTEMS FOR CONTROLLING AN ORAL CARE DEVICE**

(30) Priority: 11.11.2020 US 202063112308 P
(71) Applicant: Koninklijke Philips N.V., 5656 AG Eindhoven (NL)
(72) Inventor: VILLAR, Jose Martin, 5656 AE Eindhoven (NL); DIKMEN, Gul, 5656 AE Eindhoven (NL); KHODABAKHSHI, Fereshteh, 5656 AE Eindhoven (NL); SUKUMARAN, Prithy, 5656 AE Eindhoven (NL); HIRSCH, Kelly Laurence, 5656 AE Eindhoven (NL); FARRELL, Nathan, 5656 AE Eindhoven (NL); CHAUDHURI, Ishita, 5656 AE Eindhoven (NL); TIDBALL, Brian Esley, 5656 AE Eindhoven (NL); LABADIE LEZAMA, Ivonne, 5656 AE Eindhoven (NL); PEREIRA, Chelsea, 5656 AE Eindhoven (NL); CHOI, Peter, 5656 AE Eindhoven (NL)
(74) Representative: Philips Intellectual Property & Standards

(57) **Abstract**

The invention provides a method for providing a user with feedback on their technique when using an oral care device. The method includes recording one or more previous oral care sessions and identifying one or more oral care habits based on the recorded one or more previous oral care sessions. Feedback is generated for encouraging the user to adjust an oral care habit of the one or more oral care habits and the user is provided with the feedback during a subsequent oral care session.

## Description

### FIELD OF THE INVENTION

The invention relates to the field of oral care devices, and more specifically to the field of controlling oral care devices.

### BACKGROUND OF THE INVENTION

It is common for users of powered toothbrushes, and other oral care devices, to have, or to develop, one or more oral care habits that reduce the effectiveness of oral care sessions with said device.

For example, in the case of powered toothbrushes, users often end a tooth brushing session before the end of the recommended two minute brushing routine. In particular, some users regularly and consistently do not complete the recommended two minute brushing routine. Ending an oral care routine before reaching the recommended length of time for performing said routine can lead to incomplete oral care coverage and oral health complications.

In a further example, in the case of powered toothbrushes, users may brush too hard or brush at the incorrect angle leading to sub-optimal oral care.

Many existing powered toothbrushes have systems that indicate the passing of time through indicators of various types being activated at regular intervals, which may indicate to the user when to switch between different areas of the mouth. However, there is no provision for providing a user with personalized and detailed feedback relating to the oral care routines of the user.

There is therefore a need for a means addressing a greater range of oral care habits of the user.

### SUMMARY OF THE INVENTION

The invention is defined by the claims.

According to examples in accordance with an aspect of the invention, there is provided a method for providing a user with feedback on their technique when using an oral care device, the method comprising:
recording one or more previous oral care sessions;
identifying one or more oral care habits based on the recorded one or more previous oral care sessions;
generating feedback for encouraging the user to adjust an oral care habit of the one or more oral care habits; and
providing the user with the feedback during a subsequent oral care session.

The method provides a means of providing feedback to a user for adjusting an oral care habit identified across a plurality of oral care sessions.

By providing feedback on only one of the one or more oral care habits at a time, overwhelming the user with too much feedback may be prevented. By focusing on only one oral care habit at a time, the effectiveness of the feedback is increased and the user is more likely to make a positive alteration in response to the feedback.

In an embodiment, providing the user with the feedback comprises:
automatically providing the user with the feedback during a subsequent oral care session; or
requesting an input from the user to confirm that they wish to receive the feedback; and
if the user provides an input confirming that they wish to receive the feedback, providing the user with the feedback during a subsequent oral care session; and
if the user provides an input indicating that they do not wish to receive the feedback, or if the user does not provide an input, preventing the feedback from being provided to the user during a subsequent oral care session.

In this way, the user may be provided feedback automatically or the user is provided with a request to confirm that they wish to receive feedback, thereby ensuring that the user will always be aware of any feedback that will be provided. In this way, confusion of the user from unexpected feedback may be avoided and more effective changes in the oral care habits of the user may be achieved.

By providing the user with feedback automatically, it is ensured that the user will always be provided with feedback when a habit is identified as requiring a change.

In an embodiment, the feedback comprises one or more of:
a light signal;
a sound signal;
a tactile signal; and
an electrical signal.

In this way, the feedback may be provided to a user using any suitable means.

In an embodiment, recording an oral care session comprises automatically recording one or more of:
an oral care session time;
a motion signal representing a motion of the oral care device during the oral care session;
an orientation of the oral care device during the oral care session;
a pressure signal representing a pressure applied to the oral care device during the oral care session;
a proximity signal;
a force signal;
a light signal;
a flow signal;
a current signal; and
a sound of the oral care session.

In this way, the recordings of the previous oral care sessions may include a variety of different information and may be collected automatically as the user uses the oral care device. By automatically recording the oral care sessions, the method does not require any active input from the user in order to be effective.
In an embodiment, the one or more oral care habits comprises one or more of:
a time spent using the oral care device;
a frequency of use of the oral care device;
an orientation of the oral care device;
a pressure applied to the oral care device;
a motion pattern of the oral care device;
a brush head of the oral care device; and
a coverage area of the oral care device.

In this way, a variety of oral care habits may be the subject of feedback in order to encourage the user to adjust a number of oral care techniques.

In an embodiment, the method further comprises:
recording a subsequent oral care session;
determining whether an oral care habit has been adjusted based on the recorded subsequent oral care session; and
updating the feedback based on said determination.

In this way, the feedback may be updated based on each subsequent oral care session, thereby ensuring that the feedback to be provided to the user is relevant to the user's current oral care habits.

According to examples in accordance with an aspect of the invention, there is provided a computer program product comprising computer program code means which, when executed on a computing device having a processing system, cause the processing system to perform all of the steps of the method as described above.

According to examples in accordance with an aspect of the invention, there is provided a processing unit for providing a user with feedback on their technique when using an oral care device, wherein the processing unit is adapted to:
record a plurality of previous oral care sessions;
identify one or more oral care habits based on the recorded plurality of previous oral care sessions;
generate feedback for encouraging the user to adjust an oral care habit of the one or more oral care habits; and
generate an instruction to provide the user with the feedback during a subsequent oral care session.

In an embodiment, when generating an instruction to provide the user with feedback, the processing unit is adapted to:
automatically generate the instruction to provide the user with the feedback during a subsequent oral care session; or
request an input from the user to confirm that they wish to receive the feedback; and
if the user provides an input confirming that they wish to receive the feedback, provide the user with the feedback during a subsequent oral care session; and
if the user provides an input indicating that they do not wish to receive the feedback, or if the user does not provide an input, prevent the feedback from being provided to the user during a subsequent oral care session.

According to examples in accordance with an aspect of the invention, there is provided a system for providing a user with feedback on their technique when using an oral care device, the system comprising:
an oral care device;
the processing unit as described above;
a recording unit in communication with the processing unit adapted to record the plurality of previous oral care sessions; and
a feedback unit in communication with the processing unit adapted to provide the user with feedback.

In an embodiment, the processing unit, the recording unit and the feedback unit are incorporated into the oral care device.

In an embodiment, the oral care device comprises one or more of:
a powered toothbrush;
a powered brushing mouthpiece;
a powered flossing device; and
a powered tooth whitening device.

In an embodiment, the recording unit comprises one or more of:
a timer;
a motion sensor;
an orientation sensor;
a pressure sensor;
a proximity sensor;
a force sensor;
a light sensor;
a flow sensor;
a current sensor; and
a microphone.

In an embodiment, the processing unit is incorporated into a smart device, such as:
a smartphone;
a smartwatch; and
a smart home device,
wherein the oral care device further comprises a communication unit in communication with the processing unit.

In an embodiment,:
the feedback unit and/or the recording unit is further incorporated into the smart device;
the feedback unit and/or the recording unit is incorporated into the oral care device, wherein the feedback unit and/or the recording unit is in communication with the communication unit; or
the feedback unit and/or the recording unit is incorporated into a charging unit, wherein the charging unit is adapted to charge the oral care device.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a method for providing a user with feedback on their technique when using an oral care device;
Figure 2 shows a schematic representation of a powered toothbrush as an oral care device according to an aspect of the invention;
Figure 3 shows a schematic representation of an oral care system according to an aspect of the invention; and
Figure 4 shows a schematic representation of an oral care system according to a further aspect of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for providing a user with feedback on their technique when using an oral care device. The method includes recording one or more previous oral care sessions and identifying one or more oral care habits based on the recorded one or more previous oral care sessions. Feedback is generated for encouraging the user to adjust an oral care habit of the one or more oral care habits and the user is provided with the feedback during a subsequent oral care session.

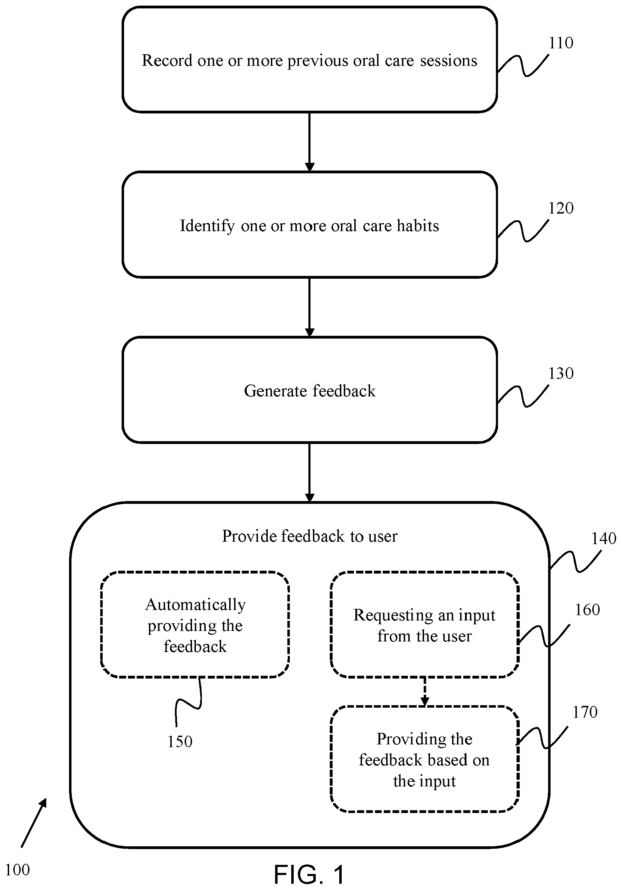

Figure 1 shows a method 100 for providing a user with feedback on their technique when using an oral care device.

The oral care device may include one or more of: a powered toothbrush; a powered brushing mouthpiece; a powered flossing device; and a powered tooth whitening device. The method of Figure 1 will be described in the context of a powered toothbrush in order to provide an exemplary illustration of how the method may be implemented in an oral care environment. However, it should be noted that the methods described herein may be applied to any of the oral care devices listed above, or indeed any suitable powered oral care device.

The method begins at step 110, by recording one or more previous oral care sessions. The one or more previous oral care sessions may include any oral care session with a given oral care device.

For example, in the case of the oral care device being a powered toothbrush, each time the user brushes their teeth using the powered toothbrush, the tooth brushing session may be recorded as an oral care session. Over multiple recorded oral care sessions, it is possible to identify the typical brushing, or oral care, habits of the given user.

For example, if the user always brushes their teeth for at least the recommended two minute time period, they may not require any further encouragement to perform the oral care routine for the recommended amount of time; however, said user may be brushing their teeth too hard or too rapidly, referred to as scrubbing, in which case it would be desirable to encourage the user to brush more softly or slower. The recording of oral care sessions may be ongoing in order to ensure that the user does not resume a habit during subsequent oral care sessions.

However, if a user does end an oral care session before the period of time has elapsed and brushes their teeth too hard, they may benefit from receiving multiple pieces of feedback to encourage them to perform the oral care routine in the most optimal way. Multiple pieces of feedback delivered at the same time, or in quick succession, may be confusing to the user. Accordingly, the feedback may be delivered to the user for one habit at a time in order to avoid confusion of the user.

Recording the plurality of previous oral care sessions may be performed in a number of ways. For example, recording an oral care time for a previous oral care session may comprise one or more of: automatically recording the oral care time by starting a timer when the oral care device is turned on and stopping the timer when the oral care device is turned off; determining the oral care time based on a motion signal obtained by a motion sensor of the oral care device; determining the oral care time based on a pressure signal obtained by a pressure sensor of the oral care device; determining the oral care time based on a proximity signal obtained by a proximity sensor of the oral care device; determining the oral care time based on a force signal obtained by a force sensor of the oral care device; determining the oral care time based on a light signal obtained by a light sensor of the oral care device; determining the oral care time based on a flow signal obtained by a flow sensor of the oral care device; determining the oral care time based on a current signal obtained by a current sensor of the oral care device; and determining the oral care time based on a sound signal obtained by a microphone in the vicinity of the oral care device.

In step 120, one or more habits are identified based on the recorded one or more previous oral care sessions.

The one or more habits may be any habit, or characteristic, relating to the use of the oral care device by the user. For example, the one or more habits that may be identified from the recorded previous oral care sessions may include one or more of: a time spent using the oral care device; a frequency of use of the oral care device; an orientation of the oral care device; a pressure applied to the oral care device; a motion pattern of the oral care device; a brush head of the oral care device; and a coverage area of the oral care device.

In a particular example, an oral care habit may include insufficient or excessive time spent performing an oral care routine. In a further example, an oral care habit may include a lower, or higher, that necessary frequency of performing a given oral care routine. In a further example, an oral care habit may include an incorrect angle or orientation of the oral care device during an oral care session. In a further example, an oral care habit may include excessive or insufficient pressure applied to the oral care device during the oral care session. In an example, an oral care habit may include a sub-optimal motion pattern, such as a scrubbing motion, when using the oral care device. In an example, an oral care habit may include an incorrect brush head for a given oral care routine. In a further example, an oral care habit may include an insufficient coverage area of the oral care routine.

It should be noted that the habit may be updated with each subsequent oral care session. For example, updating the habit may include calculating a moving average, such as a moving average time spent performing an oral care routine or a moving average pressure applied to the oral care device during oral care sessions, based on each subsequent oral care session. Further, the method may include determining whether an oral care habit has been adjusted by the user based on the recorded subsequent oral care session.

In step 130, feedback is generated for encouraging the user to adjust an oral care habit of the one or more oral care habits.

The generation of the feedback may include determining a point in time during the subsequent oral care session at which the feedback should be provided to the user. For example, it may be determined that the feedback should be provided before, or when, the average time spent using the oral care device is reached. Further, it may be determined that, when an oral care routine is performed at a certain time of day, the average oral care time may differ from other times of day when an oral care routine is performed, meaning that the feedback may be provided at different points in a subsequent oral care session based on the time of day the subsequent oral care routine is performed. In the example of the user brushing their teeth in the morning and the evening, the user may require feedback at a different point in time during the morning oral care session than in the evening oral care session.

Further, the generation of the feedback may include determining the optimal means of delivering the feedback to the user, such as by way of a light signal or a sound signal to be provided to the user in order to communicate the generated feedback.

In the case where multiple habits are identified from the one or more previously recorded oral care sessions, the generation of the feedback may include prioritizing one habit to be addressed by way of the feedback before the other habits. For example, the habits may be ranked based on a measure of the negative impact that each habit may have of the oral health of the subject. The order in which the feedback is provided to the user may be determined based on any suitable measure derived from the previously recorded oral care sessions.

The feedback, which may include the feedback order, delivery means and the like, may be updated based on a recorded subsequent oral care session. Thus, it may be ensured that the relevance of the feedback provided to the user is maintained over time.

The determination of the feedback to be provided to the user may be computed using any suitable algorithm. Further, the algorithm may be designed by providing the oral care habits to a machine learning algorithm as an input and in order to generate a recommended set of feedback as an output. Once the algorithm has been designed and loaded to the oral care device, the algorithm may become static i.e. no longer changing in response to new input data, thereby reducing the computational requirements of the oral care device.

A machine-learning algorithm is any self-training algorithm that processes input data in order to produce or predict output data. Here, the input data comprises oral care habits and the output data comprises feedback to be provided to the user.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example oral care habits. The training output data entries correspond to recommended feedback to be provided to the user.

In step 140, the feedback is provided to the user during a subsequent oral care session. The feedback may comprise one or more of: a light signal; a sound signal; a tactile signal; and an electrical signal.

The method may progress to step 150, wherein feedback may be automatically provided to the user during the subsequent, or the current, oral care session. Alternatively, the method may progress to step 160, wherein an input may be requested from the user to confirm that they wish to receive the feedback. In step 170, if the user provides an input confirming that they wish to receive the feedback, the feedback is provided to the user during the subsequent oral care session. In step 170, if the user provides an input indicating that they do not wish to receive the feedback, or if the user does not provide an input, the feedback may be prevented from being provided to the user during the subsequent oral care session.

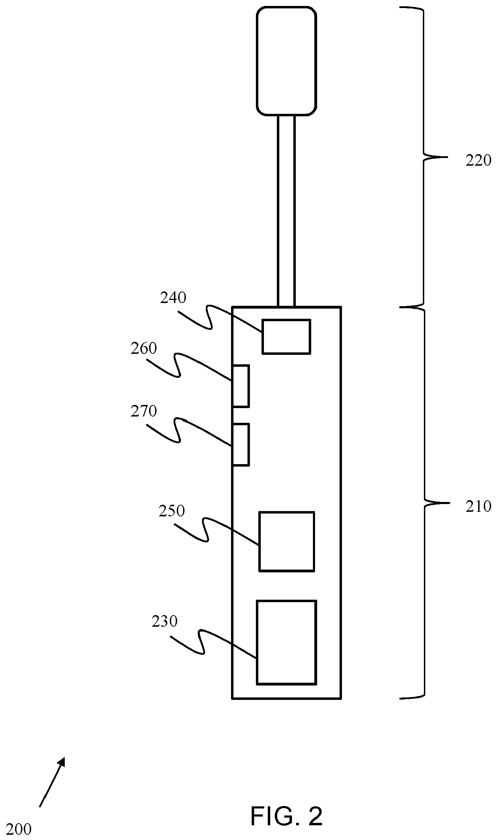

Figure 2 shows a schematic representation of a powered toothbrush 200 as an oral care device, which may form at least part of a system for providing a user with feedback on their technique when using an oral care device. The powered toothbrush may comprise a body portion 210 and a brush head portion 220, wherein the brush head portion is adapted to be provided to a user's mouth for performing an oral care routine. The body portion 210 of the powered toothbrush 200 may include a battery 230 and a drive unit 240 for operating the brush head portion 220.

In the example shown in Figure 2, the powered toothbrush 200 includes a processing unit 250 for providing a user with feedback on their technique when using an oral care device. The processing unit may be adapted to implement the methods described above with reference to Figure 1.

More specifically, the processing unit 250 may be adapted to record a plurality of previous oral care sessions; identify one or more oral care habits based on the recorded plurality of previous oral care sessions; generate feedback for encouraging the user to adjust an oral care habit of the one or more oral care habits; and generate an instruction to provide the user with the feedback during a subsequent oral care session. Further, the processing unit may be adapted to automatically generate the instruction to provide the user with the feedback during a subsequent oral care session; or request an input from the user to confirm that they wish to receive the feedback; and if the user provides an input confirming that they wish to receive the feedback, provide the user with the feedback during a subsequent oral care session; and if the user provides an input indicating that they do not wish to receive the feedback, or if the user does not provide an input, prevent the feedback from being provided to the user during a subsequent oral care session.

In the example shown in Figure 2, the powered toothbrush comprises a recording unit 260, adapted to record the plurality of previous oral care sessions, and a feedback unit 270 adapted to provide the user with the determined feedback.

The recording unit 260 may comprise one or more of: a timer; a motion sensor; a pressure sensor; a proximity sensor; a force sensor; a light sensor; a flow sensor; a current sensor; and a microphone.

For example, the recording unit may comprise a timer, wherein the timer is adapted to start timing an oral care session when the user turns the oral care device on and stop timing the oral care session when the user turns the oral care device off.

In a further example, the recording unit may comprises a motion/orientation sensor, such as an accelerometer, a gyroscope and/or an inertial measurement unit. In the case where the recording unit comprises a motion sensor, the recording unit may be adapted to begin recording an oral care session when a movement is detected that corresponds to the user interacting with the oral care device. The recording unit may stop recording the oral care session when the motion sensor no longer detects any motion for a predetermined length of time, such as 10 seconds. In the example shown in Figure 2, the motion sensor may be located at any point within the powered toothbrush. In the case that the recording unit includes a motion/orientations sensor, the oral care session recordings may include information such as a movement (brushing) pattern of the oral care device, and an angle of the oral care device, when in use.

In a further example, the recording unit may include a pressure sensor, in which case the recording unit may be adapted to begin recording an oral care session when a change in pressure is detected that corresponds to the user interacting with the oral care device. For example, if the pressure sensor is located in the brush body 210, the change in pressure may correspond to the user gripping the brush body. If the pressure sensor is located in the brush body, the pressure sensor may also detect a force applied to the brush head 220 by way of the physical connection between the brush head and the brush body.

Alternatively, if the pressure sensor is located within the brush head 220, the change in pressure may correspond to the brush head entering the user's mouth or the brush head applying pressure to the teeth of the user. In a further alternative example, if the pressure sensor is located at the base of the brush body, the change in pressure may correspond to the oral care device being picked up. The recording unit may stop recording the oral care session when the pressure sensor senses a change in pressure restoring the pressure reading to the level recorded before the session began, for example, when the user releases the oral care device, the oral care device leaves the user's mouth or the oral care device is set down. In the case that the recording unit comprises a pressure sensor, the recording of the oral care sessions may include information relating to the pressure applied to the oral care device, or the oral cavity, when the oral care device is in use.

In a further example, the recording unit may comprise a proximity sensor, in which case the recording unit may be adapted to begin recording an oral care session when it is detected that the oral care device has been brought within a given proximity of the user. For example, if the proximity sensor is located in the brush body 210, the proximity detection may be related to the detection of the hand of the user picking up the oral care device. Alternatively, if the proximity sensor is located in the brush head 220, the proximity detection may be related to the detection of the brush head entering an oral cavity of the user. The recording unit may stop recording the oral care session when the proximity sensor detects that the user is no longer within a given range of the oral care device.

In an example, the recording unit may comprise a force sensor, in which case the recording unit may be adapted to begin recording an oral care session when a force is applied to the oral care device by the user. For example, if the force sensor is located in the brush body 210, the force detection may be related to the detection of the user picking up the oral care device. Alternatively, if the force sensor is located in the brush head 220, the force detection may be related to the detection of force being applied to the brush head upon entering an oral cavity of the user. The recording unit may stop recording the oral care session when the force sensor detects that force is no longer being applied to the oral care device. In the case that the recording unit comprises a force sensor, the recording of the oral care sessions may include information relating to the force applied to the oral care device, or the oral cavity, when the oral care device is in use.

In a further example, the recording unit may comprise a light sensor, in which case the recording unit may be adapted to begin recording an oral care session when a change in light is detected. For example, if the light sensor is located in the brush body 210, the light detection may be related to the detection of the hand of the user picking up the oral care device, thereby blocking light from impacting the light sensor. Alternatively, if the light sensor is located in the brush head 220, the light detection may be related to the detection of the brush head entering an oral cavity of the user, which reduces the amount of light impacting the light sensor. The recording unit may stop recording the oral care session when the light sensor detects that the light levels return to the state prior to the oral care session beginning. Further, if the oral care device generates therapeutic light, for example for tooth whitening purposes, the light sensor may be adapted to sense when, and for how long, the therapeutic light is active.

In an example, the recording unit may comprise a flow sensor, in which case the recording unit may be adapted to begin recording an oral care session when it is detected that a flow passes through the flow sensor associated with the oral care device. For example, a flow sensor may be located in either the inflow or outflow of a sink in a room where the oral care device is being used, in which case the flow detection may be related to the detection of a tap being turned on by the user thereby causing a flow to pass through the sensor. The recording unit may stop recording the oral care session when the flow sensor detects that the flow is no longer passing through the sensor. The flow sensor may include a communications unit in order to communicate the flow sensor data with the oral care device.

In a further example, the recording unit may comprise a current sensor, in which case the recording unit may be adapted to begin recording an oral care session when it is detected that a current is flowing between two electrodes. For example, if the current sensor is located in the brush body 210, the current detection may be related to the detection of the hand of the user picking up the oral care device, the hand of the user completing a circuit with the current sensor. Alternatively, if the current sensor is located in the brush head 220, the current detection may be related to the detection of the brush head entering an oral cavity of the user, in which case the saliva of the user may complete the circuit with the current sensor. The recording unit may stop recording the oral care session when the current sensor detects that the current has dropped below a given level.

In a yet further example, the recording unit may comprise a microphone, in which case the recording unit may be adapted to begin recording an oral care session when a sound associated with the oral care session is detected. The sound may be any suitable trigger sound, such as, the sound of the driving unit or the sound of teeth being brushed. Alternatively, the user may speak a command word to be detected by the microphone, which is intended to begin the recording of the oral care session. The recording unit may stop recording the oral care session when the microphone ceases to detect the trigger sound or the user speaks a command word intended to end the recording of the oral care session.

The feedback unit 270 may comprise one or more of: a light source; a speaker; a tactile feedback unit; and an electrical signal generator.

For example, if the feedback unit comprises a light source, the feedback may comprise one or more of: a constant light; a blinking light; a change in light color; and the like. If the feedback unit comprises a speaker, the feedback may comprise one or more of: a brief audible note; an elongated audible note; a repeating audible note; a plurality of audible notes; and the like. If the feedback unit comprises a tactile feedback unit, such as a vibration unit, the feedback may comprise one or more of: a brief tactile signal; an elongated tactile signal; a repeating tactile signal; a plurality of tactile signals; and the like. If the feedback unit comprises an electrical signal generator, the feedback may, for example, comprise an electrical signal adapted to alter the behavior of the oral care device in order to provide the user with feedback. For example, the electrical signal generator may generate a signal to briefly alter the behavior of the drive unit in order to provide the user with the feedback.

If the feedback unit comprises more than one of the feedback means described above, the user may select their preferred feedback type. Further, each different feedback type may be associated with a different habit derived from the recorded oral care sessions.

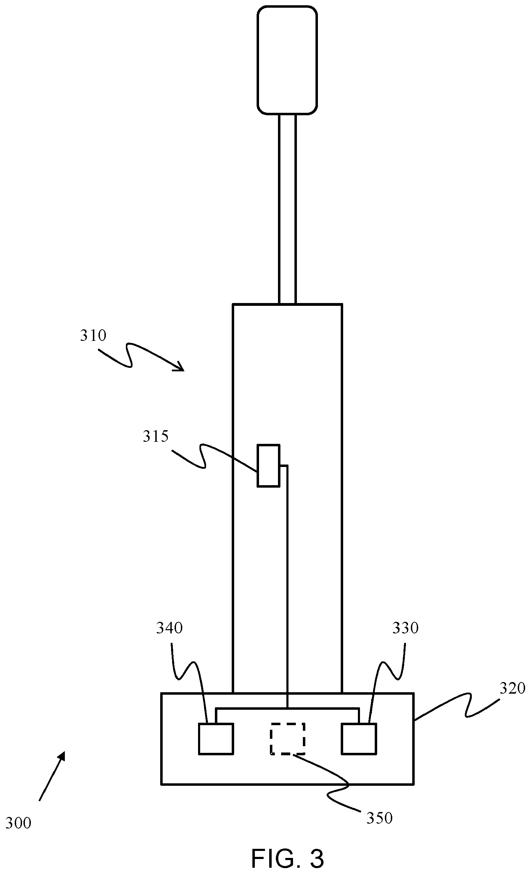

Figure 3 shows a schematic representation of an oral care system 300, wherein the oral care device 310 is a powered toothbrush comprising a recording unit 315, such as the recording units described above with reference to Figure 2.

In the example shown in Figure 3, the oral care system 300 comprises a charging unit 320 adapted to receive, and charge, the oral care device 310. Further, the charging unit 320 comprises a processing unit 330 and a feedback unit 340. The processing unit and feedback unit may be implemented as described above with reference to Figure 2. In other words, the processing unit and feedback unit may be provided in the charging unit of the oral care system rather than the oral care device itself. The recording unit of the oral care device may be brought into communication with the processing unit when the oral care device is brought into contact with the charging unit.

Alternatively, the processing unit may be provided in the oral care device and the feedback unit may be provided in the charging unit, or vice versa. The oral care device and the charging unit may comprise communication units to establish wireless communication between the oral care device and the charging unit when they are not physically connected.

Further, the charging unit may comprise a recording unit 350, such as a pressure sensor or a microphone as described above. Alternatively, the recording unit may comprise a timer adapted to start timing an oral care session when the user removes the oral care device from the charging unit and stop timing the oral care session when the user returns the oral care device to the charging unit.

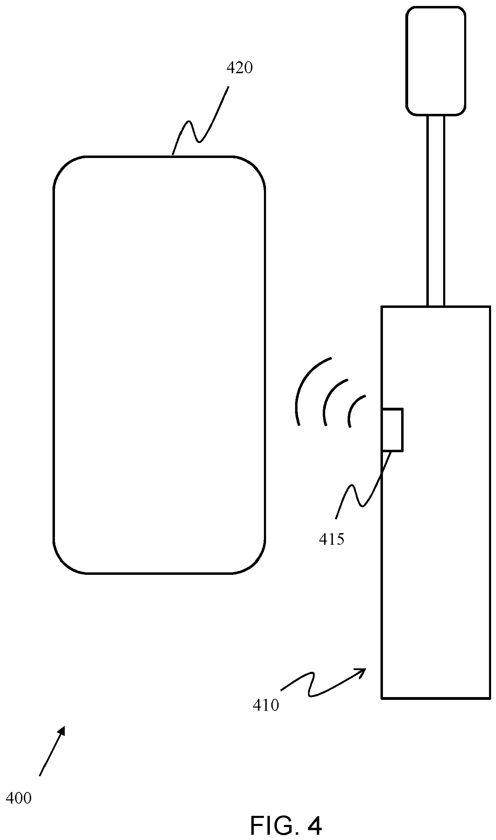

Figure 4 shows a schematic representation of an oral care system 400, wherein the oral care device 410 is a powered toothbrush comprising a communication unit 415 in wireless communication with a remote processing unit in the form of a smart device 420. It should be noted that any remote processing unit capable of communication with the communication unit may be used to perform the methods herein. For example, at least part of the remote processing unit may be incorporated into a computer, a laptop, a tablet, a server, a distributed processing network and the like.

In the example shown in Figure 4, the smart device 420 is a smartphone; however, the smart device may comprise one or more of: a smartphone; a smartwatch; and a smart home device.

One or more of the processing unit, the feedback unit and the recording unit may be incorporated into the smart device.

For example, the recording unit may be incorporated into the oral care device as described above with reference to Figure 2. The recorded oral care session may then be provided to the smart device, which includes the processing and feedback units, by way of the communication unit 415. The smart device may generate any suitable type of feedback as described above, such as: a light signal, provided by way of a display; an audible signal, provided by way of a speaker; a tactile signal, for example by generating a vibration; and an electrical signal, which may be communicated to the communication unit of the oral care device to alter a behavior of the oral care device or used to alter an aspect of the smart device to communicate the feedback to the user.

Alternatively, the smart device may include a recording unit comprising a microphone as described above. Further, if the smart device is a smart watch, the smart device may include a recording unit comprising a motion sensor.

The smart device may include a user interface adapted to provide the user with a request to confirm that they wish to receive the feedback generated by the processing unit. Further, the user interface may provide an indication of which habit the proposed feedback relates to, thereby enabling the user to correctly address the habit in response to the feedback when provided during a subsequent oral care session. In this way, the user understands why feedback in their oral care device has been triggered, thereby preventing confusion.

In an example use case, the oral care device is a powered toothbrush including a recording unit, which comprises a motion sensor and a force sensor, and the processing unit and feedback units are incorporated into a smart device in communication with the oral care device.

When the user begins using the oral care device, one or more oral care sessions are recorded and sent to the smart device to be processed. One or more oral care habits, such as scrubbing or mistiming oral care sessions, may be identified within the recordings by the processing unit of the smart device and it may be determined that the user may benefit from scrubbing feedback. The user may then be provided with a request to confirm that they wish to receive scrubbing feedback. If the user confirms that they do wish to receive the feedback, the feedback may be delivered anytime that scrubbing is detected during a subsequent oral care session.

In the case that multiple habits are detected, the user interface may provide information identifying the current habit that the user is receiving feedback for. Alternatively, different habits may be address using different types of feedback.

It should be noted that any combination of the embodiments described in Figures 2 to 4 may also be achieved. For example, the recording unit may be incorporated into the oral care device, the processing unit may be incorporated into the smart device and the feedback unit may be incorporated into the charging unit, each component being linked by one or more communication units. Further, the user interface described above may be incorporated into the oral care device or the charging units described above.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A method (100) for providing a user with feedback on their technique when using an oral care device, the method comprising:
recording (110) one or more previous oral care sessions;
identifying (120) one or more oral care habits based on the recorded one or more previous oral care sessions;
generating (130) feedback for encouraging the user to adjust an oral care habit of the one or more oral care habits; and
providing (140) the user with the feedback during a subsequent oral care session.

2. A method (100) as claimed in claim 1, wherein providing the user with the feedback comprises:
automatically (150) providing the user with the feedback during a subsequent oral care session; or
requesting (160) an input from the user to confirm that they wish to receive the feedback; and
if the user provides an input confirming that they wish to receive the feedback, providing the user with the feedback during a subsequent oral care session; and
if the user provides an input indicating that they do not wish to receive the feedback, or if the user does not provide an input, preventing the feedback from being provided to the user during a subsequent oral care session.

3. A method (100) as claimed in any of claims 1 to 2, wherein the feedback comprises one or more of:
a light signal;
a sound signal;
a tactile signal; and
an electrical signal.

4. A method (100) as claimed in any of claims 1 to 3, wherein recording an oral care session comprises automatically recording one or more of:
an oral care session time;
a motion signal representing a motion of the oral care device during the oral care session;
an orientation of the oral care device during the oral care session;
a pressure signal representing a pressure applied to the oral care device during the oral care session;
a proximity signal;
a force signal;
a light signal;
a flow signal;
a current signal; and
a sound of the oral care session.

5. A method (100) as claimed in any of claims 1 to 4, wherein the one or more oral care habits comprises one or more of:
a time spent using the oral care device;
a frequency of use of the oral care device;
an orientation of the oral care device;
a pressure applied to the oral care device;
a motion pattern of the oral care device;
a brush head of the oral care device; and
a coverage area of the oral care device.

6. A method (100) as claimed in any of claims 1 to 5, wherein the method further comprises:
recording a subsequent oral care session;
determining whether an oral care habit has been adjusted based on the recorded subsequent oral care session; and
updating the feedback based on said determination.

7. A computer program product comprising computer program code means which, when executed on a computing device having a processing system, cause the processing system to perform all of the steps of the method according to any of claims 1 to 6.

8. A processing unit (250) for providing a user with feedback on their technique when using an oral care device, wherein the processing unit is adapted to:
record a plurality of previous oral care sessions;
identify one or more oral care habits based on the recorded plurality of previous oral care sessions;
generate feedback for encouraging the user to adjust an oral care habit of the one or more oral care habits; and
generate an instruction to provide the user with the feedback during a subsequent oral care session.

9. A processing unit (250) as claimed in claim 8, wherein, when generating an instruction to provide the user with feedback, the processing unit is adapted to:
automatically generate the instruction to provide the user with the feedback during a subsequent oral care session; or
request an input from the user to confirm that they wish to receive the feedback; and
if the user provides an input confirming that they wish to receive the feedback, provide the user with the feedback during a subsequent oral care session; and
if the user provides an input indicating that they do not wish to receive the feedback, or if the user does not provide an input, prevent the feedback from being provided to the user during a subsequent oral care session.

10. A system for providing a user with feedback on their technique when using an oral care device, the system comprising:
an oral care device (200);
the processing unit (250) as claimed in any of claims 8 to 9;
a recording unit (260) in communication with the processing unit adapted to record the plurality of previous oral care sessions; and
a feedback unit (270) in communication with the processing unit adapted to provide the user with feedback.

11. A system as claimed in claim 10, wherein the processing unit (250), the recording unit (260) and the feedback unit (270) are incorporated into the oral care device (200).

12. A system as claimed in any of claims 10 to 11, wherein the oral care device comprises one or more of:
a powered toothbrush;
a powered brushing mouthpiece;
a powered flossing device; and
a powered tooth whitening device.

13. A system as claimed in any of claims 10 to 12, wherein the recording unit (250) comprises one or more of:
a timer;
a motion sensor;
an orientation sensor;
a pressure sensor;
a proximity sensor;
a force sensor;
a light sensor;
a flow sensor;
a current sensor; and
a microphone.

14. A system as claimed in claim 10, wherein the processing unit is incorporated into a smart device, such as:
a smartphone;
a smartwatch; and
a smart home device,
wherein the oral care device further comprises a communication unit in communication with the processing unit.

15. A system as claimed in claim 14, wherein:
the feedback unit and/or the recording unit is further incorporated into the smart device;
the feedback unit and/or the recording unit is incorporated into the oral care device, wherein the feedback unit and/or the recording unit is in communication with the communication unit; or
the feedback unit and/or the recording unit is incorporated into a charging unit, wherein the charging unit is adapted to charge the oral care device.
